# EUROPEAN PATENT APPLICATION

(11) **EP 4 478 374 A1**
(43) Date of publication of application: **18.12.2024**
(21) Application number: 23178862.1
(22) Date of filing: 13.06.2023
(51) Int. Cl.: G16H 40/63, G06F 3/01

(54) **USER GUIDANCE IN MEDICAL IMAGE REVIEW AND/OR ACQUISITION**

(71) Applicant: Koninklijke Philips N.V., 5656 AG Eindhoven (NL)
(72) Inventor: KUHLMANN, Daan, Eindhoven (NL); TUINHOUT, Jelle Jeroen, Eindhoven (NL); WIJN, Victor, 5656AG Eindhoven (NL)
(74) Representative: Philips Intellectual Property & Standards

(57) **Abstract**

The invention relates to providing guidance to users of medical imaging systems. In particular, a user guidance component is determined (120) based on (114) a user gaze with respect to a display that displays a medical image, (116) a medical imaging examination type or protocol and log data. In this manner, real-time and user targeted guidance can be provided by tracking the user gaze on the medical imaging system. Thereby potentially improving training of personnel of the imaging system, and reducing expensive and time-consuming trainings from experienced personnel.

## Description

### FIELD OF THE INVENTION

The invention relates to user guidance during medical image review and/or acquisition.

### BACKGROUND OF THE INVENTION

Interacting with medical imaging systems takes time to master. Whenever a new user needs to be trained or a new feature on an imaging system is released, valuable time of experts is required to train these new users and/or existent users on the new features. Currently most users are trained on the job under direct supervision or are brought up to speed during seminars.

### SUMMARY OF THE INVENTION

It is, inter alia, an object of the invention to provide user guidance during medical image review and/or acquisition. In particular, it is a desire to easily train personnel of medical imaging systems on new features and to increase the training capacity of novice users. This is achieved by using gaze tracking is providing feedback according to the gaze tracked of a user.

The invention is defined by the independent claims. Advantageous embodiments are defined in the dependent claims.

In an aspect of the invention, there is provided a computer-implemented method to provide user guidance in medical image review and/or acquisition, the method comprising:
- receiving:
   - a user gaze with respect to a display that displays a medical image;
   - a medical imaging examination type or protocol; and
   - log data; and
- determining a user guidance component based on:
   - the user gaze;
   - the received medical examination type or the type of measurement; and
   - the log data.

The inventors have noticed that according to this method, a user of for example a medical imaging system or a medical image review system may receive user specific guidance components similar to the guidance that could be received in a student-mentor relationship. The method can automatically detect on which parts of an imaging review or acquisition procedure a user struggles by tracking the users real-time gaze, and target the guidance to those struggles. The inventors have further noticed that this method may be suitable to release new features to an image review or acquisition system as the method allows to provide guidance to use this feature to the users based on user needs. For example, when a new feature in cardiac ultrasound is released, users for whom this feature may be relevant may be guided on how to use this feature during image review or acquisition in real-time.

In an example the log data comprises:
- historical data of the medical examination type/protocol or the measurement type/protocol; and/or
- historical gaze tracking data.

In an example, the method may further comprise:
- receiving a user identification;
and wherein the log data further comprises user specific log data based on the user identification.

In an example, the method may further comprise:
- receiving a user experience level;
and wherein the user guidance component is further based on the user experience level.

In an example the method may further comprise:
- receiving a user input;
and wherein the user guidance component is further based on the user input. In this manner the user may be alerted directly if the user input is expected or unexpected in view of the medical routine being performed.

In an example the method may further comprise determining a user adeptness, and based on the user adeptness the method may further comprises any one of the following steps:
- determine the user guidance component further based on the user adeptness;
- determine a confidence of the user diagnostic based on the user adeptness;
- recommend further training of the user based on the user adeptness; and
- recommend review by an expert user, based on the user adeptness.

In this manner, the guidance component may be based on the user adeptness. For example, the guidance may be different for adept or inadept users. An adept user may receive less guidance than an inadept user. Further, an adept user may receive more targeted guidance towards specific features which are not given to inadept users in order to not overwhelm inadept users. Similarly, the adept user may receive fewer guidance in order to not disrupt the user's workflow.

In an example the method may comprise:
- determining, based on the user gaze, an updated medical imaging parameter. In other words, a user guidance component such as a suggestion to modify the image settings, may be implemented automatically.

In an aspect of the invention there is provided a computer program product comprising instructions for enabling a processor to carry out any of the above methods.

In an aspect of the invention there is provided a medical imaging system comprising:
- a display configured to display a medical image;
- a gaze tracking device configured to track a user gaze on the display; and
- a processor in communication with the display and the gaze tracking device and configured to carry out any of the above methods.

In an example the display may further be configured to display the user guidance component.

In an example the medical imaging system may further comprise:
- a user interface in communication with the processor to receive a user input. For example, to receive a user experience or skill level. The user interface may for example be a button held by the user, a pedal to be used by the user, a touch screen, etc.

In an example, the user interface is further configured to output the user guidance component to the user. For example, by vibrating the user interface, or by producing a sound signal (e.g., a recorded message).

In an example the medical imaging system may further comprise:
- a user identification device in communication with the processor to identify a user.

In an example the system may further comprise:
- a medical image acquisition device in communication with the processor and configured to acquire the medical image. The medical image acquisition device may be any suitable imaging sensor such as for example an X-ray source and detector, an ultrasound probe, etc.

In an example, the medical image acquisition device is further configured to:
- determining, based on the user gaze, an updated medical imaging parameter, and
- acquire a new medical image based on the updated medical imaging parameter.

These and other aspects of the invention will be apparent from and elucidated with reference to the embodiments described hereinafter.

### BRIEF DESCRIPTION OF THE DRAWINGS

Fig. 1 shows a method according to an aspect the invention.
Fig. 2 shows a schematic of a heat mat obtained from gaze tracking of a user according to an embodiment of the invention.
Fig. 3A-B show stages during an interaction with a schematic of a display according to an embodiment of the invention.
Fig. 4A-B show stages during an interaction with a schematic of a display according to an embodiment of the invention.
Fig. 5 shows an ultrasound imaging system according to an embodiment of the invention.
Fig. 6 shows a processing circuit according to an embodiment of the invention.

### DETAILED DESCRIPTION OF EMBODIMENTS

The invention will be described herein with reference to the Figures.

It should be understood that the description and specific examples provided, while indicating exemplary embodiments, are intended for purposes of illustration only, and not intended to limit the scope of the invention. It should also be understood that the figures are mere schematics and not drawn to scale. It should also be understood that the same reference numerals are used throughout the Figures to indicate the same or similar parts.

According to the invention there is provided a method 100 to provide user guidance using gaze tracking. In particular, the method 100 comprises first step 110 of receiving various information components, based on which in a second step 120, the method 100 determines a user guidance component.

In a sub-step 114 of step 110, the method 100 receives a user gaze with respect to a display that displays a medical image. This user gaze will typically be the real-time gaze on a display that shows the medical image. The gaze may further also be with respect to other objects displayed on the display, such as but not limited to annotations on the medical image or control buttons.

In an optional sub-step of step 110, the method 100 may receive the medical image that the user is gazing at. In an example the medical image is received from a medical image acquisition system, such as an ultrasound imaging system, an X-ray imaging system, a Magnetic Resonance imaging system, a Computed Tomography imaging system, or any other medical imaging system with a suitable imaging sensor. In an example the medical imaging system is received from a data storage such as a picture archiving and communication system or a local or remote server. In an example the medical image is shown in a display. The display according to this example may in addition to the medical image also show settings in which the medical image was acquired, annotations on the medical image and/or control buttons. In an example, the control buttons may change the imaging settings of the medical imaging device.

In a sub-step 116 of step 110, the method 100 receives a medical imaging examination type or protocol. In an example, a medical imaging examination type may be a fetal ultrasound examination, such as a 20-week fetal ultrasound examination, also known as a second-trimester anomaly scan. In this examination type, typically a protocol is followed to determine the development and well-being of the fetus by performing:
1) Fetal biometry measurements, including but not limited to: Crown-Rump Length, Biparietal Diameter, Head Circumference, Abdominal Circumference, Femur Length;
2) Fetal anomaly assessment, including but not limited to examination of brain, spine, face, heart, abdomen, limbs, umbilical cord and placenta;
3) Fetal Movement observation and Amniotic Fluid measurement;
4) and in many cases, a gender identification through visual inspection of the reproductive organs.

In another example, the medical examination type or protocol may be that of a cardiac ultrasound examination to evaluate the function of a heart. In such a cardiac ultrasound examination, for example, the following views may be obtained: Parasternal Long-Axis View, Parasternal Short-Axis View, Apical Four-Chamber View, Apical Two-Chamber View, Apical Three-Chamber View and Subcostal View. In some protocols the order in which to acquire certain views or take certain measurements may be pre-determined, while in other examination types, the user is free to alternate the order.

In a sub-step 118 of step 110, method 100 receives log data. This log data may comprise data of previous examinations. In other words, this log data may comprise a medical image, a medical examination type or protocol, and a user gaze from at least one previous medical examination. For sake of clarity, the log data may thus also be addressed to as a historical medical image, historical data of a medical imaging examination type or protocol and historical gaze tracking data. In an example the log data is user specific. In other words, the method 100 receives information regarding previously carried out examinations of the current user. In an example the log data is specific to the machine used for image acquisition. In an example, the log data is specific to the machine used for image review. In an example, the log data may comprise any combination of the previous. In other words, log data of the current user, log data of the machine used for image acquisition, log data of the machine used for image review, or any combination thereof.

In optional embodiments, the method 100 may further comprise a step of user identification. In an example the user identification may be from a user identification device, such as a card reader, or through user log-in. Identifying the user enables the method 100 to retrieve user specific data, such as user specific log data. By using user specific log data, the method 100 may further be optimized for each user, taking into account user preferences, such as a particular order for a medical imaging examination type. For example, in the fetal ultrasound examination discussed above, some users may alter the order of the steps. For example, a particular user may perform the second-trimester anomaly scan in the following order:
1) Fetal Movement observation and Amniotic Fluid measurement;
2) Fetal anomaly assessment, including but not limited to examination of brain, spine, face, heart, abdomen, limbs, umbilical cord and placenta;
3) Fetal biometry measurements, including but not limited to: Crown-Rump Length, Biparietal Diameter, Head Circumference, Abdominal Circumference, Femur Length;
4) and in many cases, a gender identification through visual inspection of the reproductive organs.

Thus, by including these user specific preferences through the inclusion of user specific log data, the method 100 can be optimized to improve the user experience and minimize redundant and to some users annoying user guidance components.

In an example, as the user is identified, the method 100 may directly retrieve or receive a user experience level from a user data base. Alternatively or in addition, the method 100 may ask the user to input a particular user experience level, e.g., beginner, intermediate, expert, via a user interface (e.g., a button held by the user, a pedal on the floor, a mouse, a keyboard, a touch surface, etc.). Based on the user experience level, the user guidance components may be adjusted. For example, a user classified as beginner may receive more elaborate and more frequent user guidance components than a more experienced user.

In step 120, the method 100 determines a user guidance component based on the information or data received in step 110. In particular, the user guidance component is based on the tracked user gaze, the received medical examination type or protocol and the received log data, and optionally on the identified user and user experience level.

According to an embodiment, the method 100 compares the tracked user gaze (current gaze or real-time gaze) with the log data user gaze (historic gaze) for the same medical examination. In this manner, the method 100 can determine whether a medical imaging examination type or protocol is followed. Based on the comparison of current and historic gaze, the method 100 may determine whether the speed of the current examination is appropriate, e.g., too fast, too slow or adequate. Depending on the outcome the method 100 may then provide a user guidance component indicating to the user to slow down or speed up. In case the method 100 detects that a user is stuck or struggling at a particular step, for example because the gaze is dwelling at the same spot, the method 100 may provide a user guidance component indicating the next steps in the medical imaging examination or protocol. This user guidance component may be a single suggestion or a plurality of options with which the user may continue.

The user guidance component may further be dependent on the experience, skill level or adeptness of the user. For example, an experienced or skilled user may receive less guidance and/or receive guidance less frequently than a less experienced user than a novice user. For example, the system assumes the user is aware of how to change the depth in an ultrasound machine and will not recommend optimization. In an example for experienced or skilled users, the user guidance component may focus on the user's particular needs. For example, the system may recognize a user that frequently performs a particular type of exam for which unique and specific settings exist. Method 100 may in this case recommend user targeted guidance component. In an example for a novice, inexperienced or inadept users, the user guidance component may only include guidance to frequently used settings and pre-sets. For example, the system may act quickly to recommend to adjust settings such as the ultrasound depth, gain, field of view, etc., but the system may not recommend settings used infrequently.

In an optional embodiment, the method 100 may receive a user experience, skill or adeptness level. For example, the user experience level may be queried from the user before initiating image review or acquisition. For example, the user is asked to input on a screen or select from a list of options on a screen an experience or expertise level (e.g., beginner/intermediate/expert). In an example the user experience level may be extracted from a database based on the user identification.

Alternatively or additionally, the method 100 may determine a user skill level or adeptness. The user adeptness may be determined by comparing the current gaze with the historic gaze of the same and/or of different users. For example, by assessing whether the current user gaze moves directly, quickly or swiftly between the expected gaze locations for a medical imaging review or acquisition procedure. The user adeptness may also be determined based on the settings and/or pre-sets used by the user. For example, a user that is detected never to optimize the depth parameter may be considered a less experienced user.

Based on the determined user adeptness, each user may be assessed in terms of user adeptness with respect to their historical prior use and/or other users. Based on the determined user adeptness, the method 100 may further include a step to recommend or suggest further training. This recommendation or suggestion may be a direct to the user, a superior or to a central system where the relevant personnel receives such a notification and considers a follow-up action.

In an example, based on the determined user adeptness, the system may further recommend to have the result reviewed by a more expert user. For example, the system may have detected hesitation of the user during the image review or image acquisition, which may be indicative of low user confidence in his/her own conclusion. In such an event the system may thus include an automatic quality control by recommending additional review. In an example, the method 100 may determine a confidence of a user diagnostic result at the end of the imaging review or acquisition procedure based on the user adeptness.

According to an embodiment, the method 100 may further include a step of receiving a user input. For example, a user may select different imaging parameters. Based on the user input, the method 100 may determine a user guidance component alerting the user that a selection is contrary to what is expected in a particular medical routine.

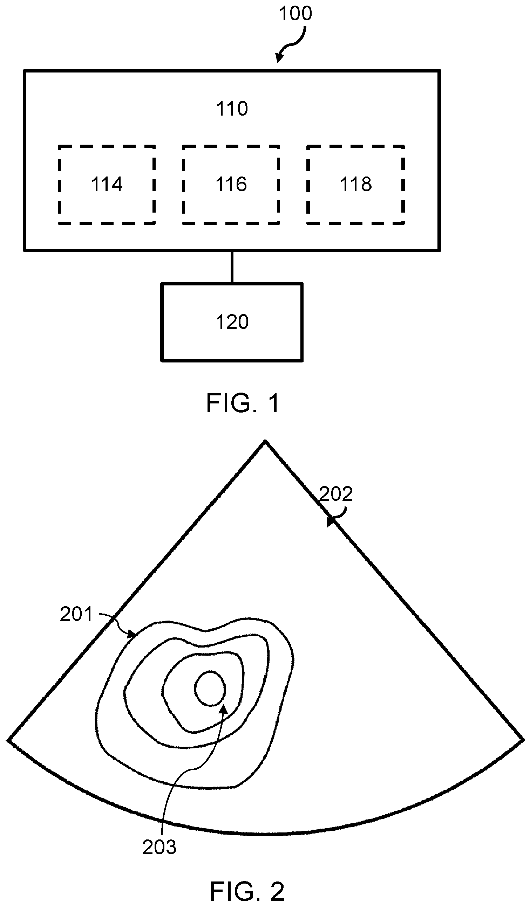

According to an embodiment, the method 100 may in addition to providing the user guidance component, also determine an updated medical imaging parameter. For example, the method 100 may identify a long gaze at a particular anatomical feature in the medical image, and the method 100 may automatically determine improved medical imaging parameters to improve the focus (e.g., gain, brightness, depth, zoom, etc.) of the medical image at that anatomical feature. In an example, the determined medical imaging parameter may also be implemented automatically, for example to auto-focus at the gazed at anatomical feature. In a preferred implementation the area in which to improve focus is determined by generating a heat map 201 of the user gaze on the medical image 202 as shown in Fig. 2. In the region where the heat map 201 shows the highest concentration of user gaze, viz. region 203, the image may then for example auto-focus by adjusting the imaging parameters (e.g., brightness, depth, gain, zoom in/out, beam steering, etc.). In this manner, the optimal field of view can be given to the user without requiring any interaction. However, it should be clear that this autofocus may also be a recommendation to the user that the user then has to accept or reject.

According to an embodiment, the method 100 may further include a step of assessing the productivity of a user. For example, the method 100 may track the average medical imaging examination type of a user over the duration of a shift and compare this to the historical data of that same user. Based on this comparison, the method 100 may then recommend a break to the user due to declining productivity or increased chance of mistakes.

A user guidance component according to method 100 may be of any kind, including but not limited to visual guiding components that appear on a screen, tactile guidance components (e.g., vibration of a controller) or audible guidance components. In an example these visual guiding components may be particular geometries such as arrows, circles, crosses, squares, etc. to guide the user. For example, a target gaze at a particular anatomical feature or control button may be displayed with an optional arrow from the users gaze to the target gaze. In an example the visual guiding components may be pop-up screens with written instructions. For example, instructions for improved image settings, instructions for the next steps in an imaging exam, etc. These visual guiding components may appear and stay static on the display. In examples, these visual guiding components may blink or change color to catch the user's attention. Once the user's attention is attained, which can be verified by the user's gaze, the blinking or changing color may stop. In a particular example, the guiding component that appears on the screen may be a coffee cup to signal it is time for a break.

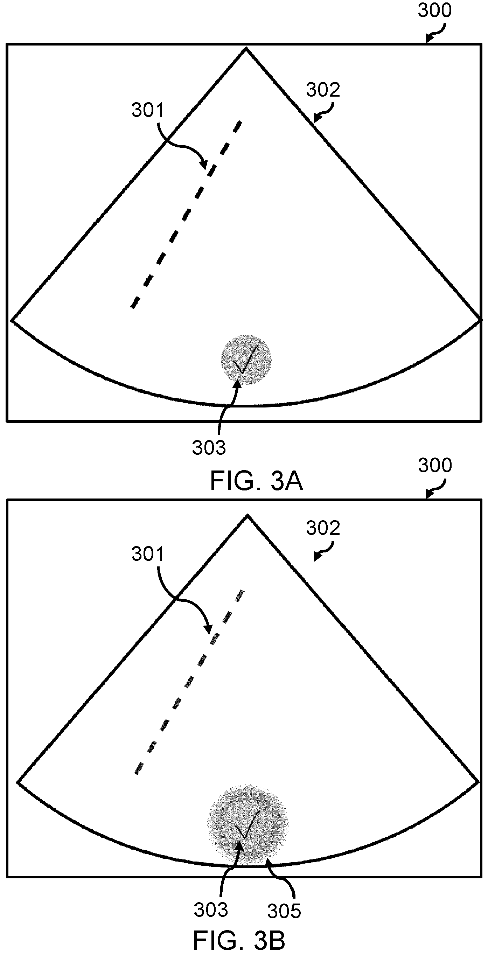

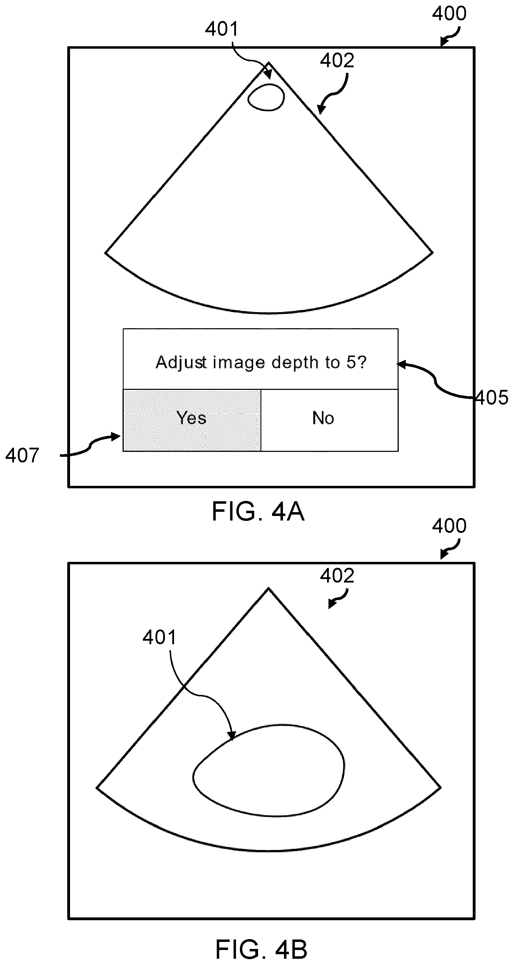

Examples of guidance components are given in Fig. 3A-B and Fig. 4A-B.

Fig. 3A displays a schematic of a display screen 300 displaying a medical ultrasound image 302 in which a measurement 301 was performed. Once the measurement is performed, the user interface asks the user to confirm the measurement by selecting the check mark 303. If the method 100 detects the user is not selecting the check mark 303 within a given time threshold, a user guidance component 305 as shown in Fig. 3B may be displayed highlighting the check mark 303. Once the user then selects the check mark, the next step in the imaging review or acquisition routine follows. It is envisioned by the invention that the user may select the check mark with any available user interface, including but not limited to a mouse, a touchscreen, a keyboard and gaze selection.

Fig. 4A displays a schematic of a display screen 400 displaying a medical image 402. In this example, the user may be gazing at a feature 401 on the display. The method 100 may determine that the user's gaze near feature 401 has exceeded a particular threshold and the method 100 may further receive or acquire an indication that the visibility of feature 401 or the region comprising feature 401 may be improved by adjusting the depth to 5. In other words, changing for example the field of view of feature 401. In this example, the method 100 thus presents a pop-up screen 405 with a recommendation to adjust the depth to 5. The user may accept or decline the recommendation by selecting either yes or no. If the user accepts the recommendation, which may be accepted by maintaining the gaze over the "yes" button 407 on the user interface, the depth of the imaging system may be adjusted giving a better view and field of view of feature 401 as shown in Fig. 4B. It is envisioned by the invention that the user may select the check mark with any available user interface, including but not limited to a mouse, a touchscreen, a keyboard and gaze selection.

In addition or replacement of visual guiding components, the guiding components may also be tactile or audible. For example, a controller held by the user may vibrate or an audible instruction may be communicated via a speaker.

The method 100 is designed to be suitable in training facilities, where training users can be trained on the spot given the user guidance component, thereby reducing the need for experienced users to intervene. For example, in a computer room where computers are equipped with gaze tracking equipment and configured to carry out method 100, a new sonographer may be trained on each computer by tracking their gaze and actions. The method 100 is also designed to be suitable for deployment in medical care settings, such as in a hospital or a general practitioners office. Here users may be helped in their medical imaging examinations based on their own expertise, potentially increasing the quality of the obtained images. The method 100 is particularly suitable when new features, such as new controls, are introduced. The method 100 may for example highlight these features by blinking on a display, to bring these to the user's attention if these features are not used in situations where these should be used.

According to an embodiment of the invention, the method 100 may be implemented with the use of machine learning (ML) or artificial intelligence (AI).

In a first step, an AI may be trained via supervised learning with training data set comprising:
- a plurality of recorded medical imaging examinations, preferably carried out by experienced personnel, wherein the recordings comprise:
- the medical examination type or protocol
- the medical images; and
- the gaze of the user carrying out the medical examination.

The training data set is then split into an input data set comprising: the medical examination type or protocol and the medical images; and an output data set comprising: the gaze of the user. In this manner the AI can learn the relationship between the medical image and medical examination type or protocol and the user gaze. Given a new medical image and medical examination type or protocol, the AI can thus learn to predict where the gaze should be. In addition, the AI may be trained to also learn imaging configuration settings, measurements that should be taken, etc.

In a second step, the AI may now further be trained or tuned to not provide its predicted gaze, but rather to let the user perform a medical imaging exam, and only when the difference between the live-gaze and predicted gaze exceeds a particular threshold, wherein the threshold may be both distance- and time-based, the AI highlights its prediction. In this manner be trained to not intervene if the user actions are in line with AI predicted gazes and only to intervene when the user actions disagree with the AI predicted gazed. The level of intervention may be tuned via thresholding or other means to best fit a particular user experience, skill level or adeptness.

The guiding capabilities of the AI may further be optimized to include a user guidance component through re-enforcement learning and/or policy learning. For example, every time the AI provides a target gaze, a user annotates the result to also provide additional guidance components to the user.

While a particular training procedure has been explained, other training procedures may be used to achieve the results intended by method 100. For example, the method may be hard coded.

The method 100 as described above may be implemented in any system wherein a user or operator interacts with a display via gaze. In an embodiment, method 100 is implemented in a medical imaging review system. For example, method 100 may be implemented in a computer connected to a picture archiving and communication system. In another embodiment, method 100 in implemented on any medical imaging acquisition system including but not limited to ultrasound systems, X-ray systems, Magnetic Resonance systems, Computed Tomography systems, or any other medical imaging systems with a suitable imaging sensor.

The method 100 is particularly suitable for ultrasound systems. Ultrasound examinations are frequently carried out during surgery in sterile environment, such that minimizing user-device interactions is advantageous to reduce sterilization needs and free up hands of the user (e.g., sonographer, clinician, doctor or otherwise operator of the ultrasound system). For example, during ultrasound guided needle placement, the user of the ultrasound machine may have both hands occupied, one holding the probe, and one holding and inserting the needle. Thus, reducing the capabilities of interacting directly with the ultrasound imaging system. In such cases, interaction via gaze tracking as described in relation to method 100 provides an efficient alternative of user-device interaction.

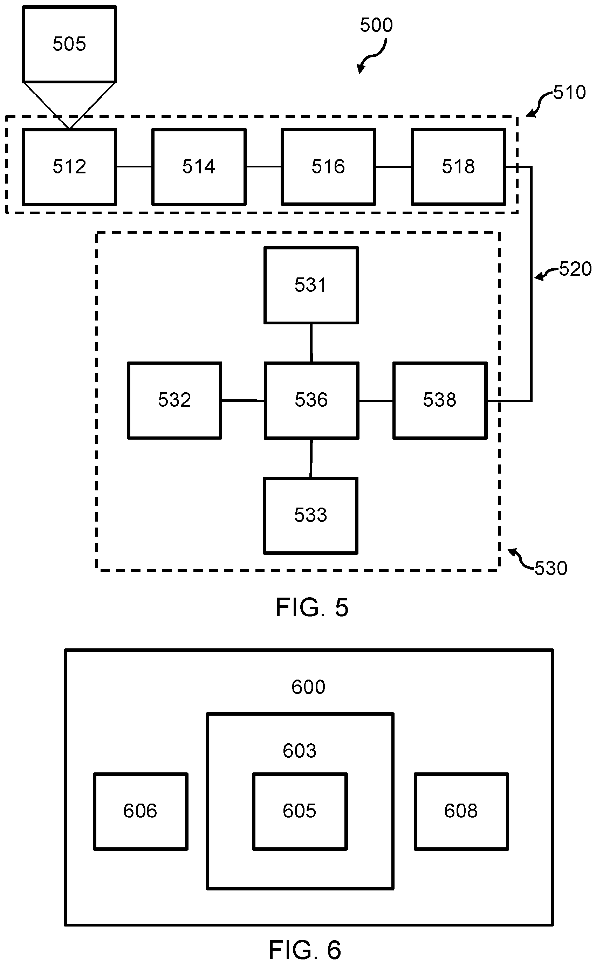

For illustrative purposes, an exemplary ultrasound system including method 100 will be described next with reference to Fig. 5. The system 500 includes an ultrasound imaging probe 510 in communication with a host 530 over a communication interface or link 520. The probe 510 may include a transducer array 512, a beamformer 514, a processor 516, and a communication interface 518. The host 530 may include a display 531, a gaze tracking device 532, a processor 536, a communication interface 538, a memory 533. The host 530 and/or the processor 536 of the host 530 may also be in communication with other types of systems or devices in replacement or addition to the here mentioned systems such as, an external memory, external display, a gaze tracking device, a subject tracking system, an inertial measurement unit, etc. It is understood that the beamformer may also be a microbeamformer. It is further understood that the components as shown here may also be configured in alternate arrangements. For example, the processor 516 and/or the beamformer 514 may be located outside of the probe 510 and/or the display 531 and/or memory 533 may be located outside of the host 530.

In some embodiments, the probe 510 is an external (i.e., non-invasive) ultrasound imaging device including a housing configured for handheld operation by a user. The transducer array 512 can be configured to obtain ultrasound data while the user grasps the housing of the probe 510 such that the transducer array 512 is positioned adjacent to or in contact with a patient's skin. The probe 510 is configured to obtain ultrasound data of anatomy within the patient's body while the probe 510 is positioned outside of the patient's body.

In other embodiments, the probe 510 can be an internal ultrasound imaging device and may comprise a housing configured to be positioned within a patient's body, including the patient's coronary vasculature, peripheral vasculature, esophagus, heart chamber, or other body or body cavity. In some embodiments, the probe 510 may be an intravascular ultrasound (IVUS) imaging catheter or an intracardiac echocardiography (ICE) catheter. In other embodiments, probe 510 may be a transesophageal echocardiography (TEE) probe. Probe 510 may be of any suitable form for any suitable ultrasound imaging application including both external and internal ultrasound imaging.

The transducer array 512 emits ultrasound signals towards an anatomical object 505 of a patient and receives echo signals reflected from the object 505 back to the transducer array 512. The transducer array 512 can include any suitable number of acoustic elements, including one or more acoustic elements and/or a plurality of acoustic elements. In some instances, the transducer array 512 includes a single acoustic element. In some instances, the transducer array 512 may include an array of acoustic elements with any number of acoustic elements in any suitable configuration. For example, the transducer array 512 can include between 1 acoustic element and 10000 acoustic elements, including values such as 2 acoustic elements, 4 acoustic elements, 36 acoustic elements, 64 acoustic elements, 128 acoustic elements, 500 acoustic elements, 812 acoustic elements, 1000 acoustic elements, 3000 acoustic elements, 8000 acoustic elements, and/or other values both larger and smaller. In some instances, the transducer array 512 may include an array of acoustic elements with any number of acoustic elements in any suitable configuration, such as a linear array, a planar array, a curved array, a curvilinear array, a circumferential array, an annular array, a phased array, a matrix array, a one-dimensional (1D) array, a 1.x-dimensional array (e.g., a 1.5D array), or a two-dimensional (2D) array. The array of acoustic elements (e.g., one or more rows, one or more columns, and/or one or more orientations) can be uniformly or independently controlled and activated. The transducer array 512 can be configured to obtain one-dimensional, two-dimensional, and/or three-dimensional images of a patient's anatomy. In some embodiments, the transducer array 512 may include a piezoelectric micromachined ultrasound transducer (PMUT), capacitive micromachined ultrasonic transducer (CMUT), single crystal, lead zirconate titanate (PZT), PZT composite, other suitable transducer types, and/or combinations thereof.

The beamformer 514 is coupled to the transducer array 512. The beamformer 514 controls the transducer array 512, for example, for transmission of the ultrasound signals and reception of the ultrasound echo signals. In some embodiments, the beamformer 514 may apply a time-delay to signals sent to individual acoustic transducers within an array in the transducer array 512 such that an acoustic signal is steered in any suitable direction propagating away from the probe 510. The beamformer 514 may further provide image signals to the processor 516 based on the response of the received ultrasound echo signals. The beamformer 514 may include multiple stages of beamforming. The beamforming can reduce the number of signal lines for coupling to the processor 516. In some embodiments, the transducer array 512 in combination with the beamformer 514 may be referred to as an ultrasound imaging component. The beamformer 514 may also be a microbeamformer.

The processor 516 is coupled to the beamformer 514. The processor 516 may also be described as a processor circuit, which can include other components in communication with the processor 516, such as a memory, beamformer 514, communication interface 518, and/or other suitable components. The processor 516 is configured to process the beamformed image signals. For example, the processor 516 may perform filtering and/or quadrature demodulation to condition the image signals. The processor 516 and/or 534 can be configured to control the array 512 to obtain ultrasound data associated with the object 505.

The communication interface 518 is coupled to the processor 516. The communication interface 518 may include one or more transmitters, one or more receivers, one or more transceivers, and/or circuitry for transmitting and/or receiving communication signals. The communication interface 518 can include hardware components and/or software components implementing a particular communication protocol suitable for transporting signals over the communication link 520 to the host 530. The communication interface 518 can be referred to as a communication device or a communication interface module.

The communication link 520 may be any suitable communication link. For example, the communication link 520 may be a wired link, such as a universal serial bus (USB) link or an Ethernet link. Alternatively, the communication link 520 may be a wireless link, such as an ultra-wideband (UWB) link, an Institute of Electrical and Electronics Engineers (IEEE) 802.11 WiFi link, or a Bluetooth link.

At the host 530, the communication interface 538 may receive the image signals. The communication interface 538 may be substantially similar to the communication interface 518. The host 530 may be any suitable computing and display device, such as a workstation, a personal computer (PC), a laptop, a tablet, or a mobile phone.

The processor 536 is coupled to the communication interface 538. The processor 536 may also be described as a processor circuit, which can include other components in communication with the processor 536, such as the memory 533, the communication interface 538, and/or other suitable components. The processor 536 can be configured to generate image data from the image signals received from the probe 510. The processor 536 can apply advanced signal processing and/or image processing techniques to the image signals. An example of image processing includes conducting a pixel level analysis to evaluate whether there is a change in the color of a pixel, which may correspond to an edge of an object (e.g., the edge of an anatomical feature). In some embodiments, the processor 536 can form a three-dimensional (3D) volume image from the image data. In some embodiments, the processor 536 can perform real-time processing on the image data to provide a streaming video of ultrasound images of the object 505.

The memory 533 is coupled to the processor 536. The memory 533 can be configured to store patient information, measurements, data, or files relating to a patient's medical history, history of procedures performed, anatomical or biological features, characteristics, or medical conditions associated with a patient, computer readable instructions, such as code, software, or other application, as well as any other suitable information or data. The memory 533 may be located within the host 530. There may also be an additional external memory, or an external memory in replacement of memory 533. An external memory may be a cloud-based server or an external storage device, located outside of the host 530 and in communication with the host 530 and/or processor 536 of the host via a suitable communication link as disclosed with reference to communication link 520. Patient information may include measurements, data, files, other forms of medical history, such as but not limited to ultrasound images, ultrasound videos, and/or any imaging information relating to the patient's anatomy. The patient information may include parameters related to an imaging procedure such a probe position and/or orientation.

The display 531 is coupled to the processor 536. The display 531 may be a monitor or any suitable display or display device. The display 531 is configured to display the ultrasound images, image videos, and/or any imaging information of the object 505.

The gaze tracking device 532 is coupled to the processor 536 and designed to detect and monitor the direction of an individual's gaze on the display 531 in real-time.

A typical gaze tracking device employs one or multiple cameras, configured to capture images of the user's eyes, and one or more infrared (IR) illuminators that emit IR light towards the user's eyes. The IR light is reflected off the cornea, forming bright pupil effects, and is captured by the camera module. This reflected IR light is then used to identify pupil centers and corneal reflections. Based on the position of the pupil centers and corneal reflections, gaze tracking software may determine a vector of the user's gaze, thus determining what the user is looking at.

To ensure accurate gaze tracking even when the user's head moves, the gaze tracking device may include a head tracking module. This module may use cameras, accelerometers, gyroscopes or the like to detect the position and orientation of the user's head. Gaze tracking software can use this head tracking data to adjust the calculated gaze vector based on the user's head movements.

The system 500 may be used to assist a sonographer or operator in performing an ultrasound scan. The scan may be performed in a point-of-care setting. In some instances, the host 530 is a console or movable cart. In some instances, the host 530 may be a mobile device, such as a tablet, a mobile phone, or portable computer. During an imaging procedure, the ultrasound system 500 can acquire an ultrasound image of a region of interest of a subject.

Fig. 6 is a schematic diagram of a processor circuit. The processor circuit 600 may be implemented in the probe 510 and/or the host system 530 of Fig. 3, or any other suitable location, such as an external computing system separate from an image acquisition devise or system. For example, an imaging sensor such as an ultrasound probe, an X-ray device (including X-ray source and X-ray detector), a camera, etc. One or more processor circuits can be configured to carry out the operations described herein. The processor circuit 600 can be part of the processor 516 and/or processor 536, or the processor circuit 600 may be separate circuit. In an example, the processor circuit 600 may be in communication with the transducer array 512, beamformer 514, communication interface 518, communication interface 538, memory 533 and/or the display 531, as well as any other suitable component or circuit within ultrasound system 500. As shown, the processor circuit 600 may include a processor 606, a memory 603, and a communication module 608. These elements may be in direct or indirect communication with each other, for example via one or more buses. The processor circuit may further be in communication with an external storage and/or to an external display. The external storage may be used to retrieve or store data, including images, ultrasound data and/or patient information. The external display may be used to display outputs and/or to control the processing circuit, for example by giving instructions.

A processor 516, 536, 606 as envisaged by the present disclosure may include a central processing unit (CPU), a graphical processing unit (GPU), a digital signal processor (DSP), an application specific integrated circuit (ASIC), a controller, a field programmable gate array (FPGA) device, another hardware device, a firmware device, or any combination thereof configured to perform the operations described herein. A processor 516, 536, 606 may also be implemented as a combination of computing devices, e.g., a combination of a DSP and a microprocessor, a plurality of microprocessors, one or more microprocessors in conjunction with a DSP core, or any other such configuration. The processor 606 may also implement various deep learning networks, which may include a hardware or a software implementation. The processor 606 may additionally include a preprocessor in either hardware or software implementation.

A memory 518, 538, 608 as envisaged by the present disclosure may be any suitable storage device, such as a cache memory (e.g., a cache memory of the processor 516, 536, 606), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. The memory may be distributed among multiple memory devices and/or located remotely with respect to the processing circuit. In an embodiment, the memory 603 may store instructions 605. The instructions 605 may include instructions that, when executed by a processor 516, 536, 606, cause the processor 516, 536, 606 to perform the operations described herein with reference to the probe 510 and/or the host 530.

Instructions 605 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. Instructions 605 may be in the form of an executable computer program or script. For example, routines, subroutines and/or functions may be defined in a programming language including but not limited to C, C++, C#, Pascal, BASIC, API calls, HTML, XHTML, XML, ASP scripts, JavaScript, FORTRAN, COBOL, Perl, Java, ADA, .NET, and the like. In particular, according to the present disclosure, instructions as disclosed with reference to method 100 are envisaged herein.

The communication module 608 can include any electronic circuitry and/or logic circuitry to facilitate direct or indirect communication of data between the processor circuit 600, the probe 510, the host 530, and/or the display 531. In that regard, the communication module 608 can be an input/output (I/O) device. In some instances, the communication module 608 facilitates direct or indirect communication between various elements of the processor circuit 600, the probe 510 and/or the host 530.

An aspect of the invention provides a computer program product comprising instructions for enabling a processor to carry out an embodiment of the above method.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

## Claims

1. A computer-implemented method (100) to provide user guidance in medical image review and/or acquisition, the method comprising:
- receiving (110):
- (114) a user gaze with respect to a display (300, 400, 531) that displays a medical image (202, 302, 402);
- (116) a medical imaging examination type or protocol; and
- (118) log data; and
- determining (120) a user guidance component (305, 405) based on:
- the user gaze;
- the received medical examination type or the type of measurement; and
- the log data.

2. The method of claim 1, wherein the log data comprises:
- historical data of the medical examination type/protocol or the measurement type/protocol; and/or
- historical gaze tracking data.

3. The method of any of the preceding claims, further comprising:
- receiving a user identification;
- and wherein the log data further comprises user specific log data based on the user identification.

4. The method of any of the preceding claims, further comprising:
- receiving a user experience level;
- and wherein the user guidance component is further based on the user experience level.

5. The method of any of the preceding claims, further comprising:
- receiving a user input;
- and wherein the user guidance component is further based on the user input.

6. The method of any of the preceding claims, further comprising determining a user adeptness, and wherein based on the user adeptness, the method further comprises any one of the following steps:
- determine the user guidance component further based on the user adeptness;
- determine a confidence of the user diagnostic based on the user adeptness;
- recommend further training of the user based on the user adeptness; and
- recommend review by an expert user, based on the user adeptness.

7. The method of any of the preceding claims, further comprising:
- determining, based on the user gaze, an updated medical imaging parameter.

8. A computer program product comprising instructions for enabling a processor to carry out the method of any of the preceding claims.

9. A medical imaging system (500) comprising:
- a display (300, 400, 531) configured to display a medical image (202, 302, 402);
- a gaze tracking device (532) configured to track a user gaze on the display; and
- a processor (516, 536, 600) in communication with the display and the gaze tracking device and configured to carry out the method of any of claims 1 to 6.

10. The system of claim 9, wherein the display is further configured to display the user guidance component.

11. The medical imaging system of any of claims 9 to 10 further comprising:
- a user interface in communication with the processor to receive a user input.

12. The system of claim 11, wherein the user interface is further configured to output the user guidance component to the user.

13. The medical imaging system of any of claims 9 to 12, further comprising:
- a user identification device in communication with the processor to identify a user.

14. The system of any of claims 8 to 12, further comprising:
- a medical image acquisition device (510) in communication with the processor and configured to acquire the medical image.

15. The system of claim 14, wherein the medical image acquisition device is further configured to:
- determining, based on the user gaze, an updated medical imaging parameter, and
- acquire a new medical image based on the updated medical imaging parameter.
